# EUROPEAN PATENT SPECIFICATION

(11) **EP 1 077 080 B1**
(45) Date of publication and mention of the grant of the patent: **22.11.2006**
(21) Application number: 99306525.9
(22) Date of filing: 18.08.1999
(51) Int. Cl.: B01J 23/68, B01J 23/89, C07D 307/08, C07C 29/17

(54) **Improved catalysts for the hydrogenation of maleic acid to 1,4-butanediol**
Verbesserte Katalysatoren zur Hydrierung von Maleinsäure zu 1,4-Butandiol
Catalyseurs améliorés pour l'hydrogénation de l'anhydride maléique en 1,4-butanediol

(43) Date of publication of application: 21.02.2001
(73) Proprietor: INEOS USA LLC, Lisle, IL 6053 (US)
(72) Inventor: Attig, Thomas G., Aurora, Ohio 44202 (US); Dubbert, Robert A., Solon, Ohio (US); Budge, John R., Beachwood, Ohio 44122 (US)
(74) Representative: Preece, Michael

(56) References cited:
- EP-A- 0 662 343
- EP-A- 0 848 991
- EP-A- 0 919 530
- WO-A-92/02298
- US-A- 5 473 086

## Description

### BACKGROUND OF THE INVENTION

### Field of the Invention

This invention relates to an improved catalyst for the hydrogenation of maleic acid, maleic anhydride or other hydrogenatable precursor to 1,4-butanediol and tetrahydrofuran. The catalyst consists of palladium, silver, rhenium and at least one of aluminum, cobalt and mixtures thereof, all on a carbon support. The use of this catalyst in processes for the hydrogenation of maleic acid, maleic anhydride or other hydrogenatable precursor to 1,4-butanediol and tetrahydrofuran is characterized by higher overall activity to reaction products and by higher yields of 1,4-butanediol with minimal formation of gamma-butyrolactone by-products.

### Description of the Prior Art

It is well known that tetrahydrofuran, gamma-butyrolactone and 1,4-butanediol are obtained by the catalytic hydrogenation of maleic anhydride and related compounds. Tetrahydrofuran is a useful solvent for natural and synthetic resins and is a valuable intermediate in the manufacture of a number of chemicals and plastics. Gamma-butyrolactone is an intermediate for the synthesis of butyric acid compounds, polyvinylpyrrolidone and methionine. Gamma-butyrolactone is a useful solvent for acrylate and styrene polymers and also a useful ingredient of paint removers and textile assistants. 1,4-butanediol (a.k.a. 1,4-butylene glycol) is useful as a solvent, a humectant, an intermediate for plasticizers and pharmaceuticals, a cross-linking agent for polyurethane elastomers, a precursor in the manufacture of tetrahydrofuran, and is used to make terephthalate plastics.

Of particular interest in the instant invention are hydrogenation catalysts comprising palladium, silver, rhenium and at least one of aluminum, cobalt and mixtures thereof, all on a carbon support, which are useful for the hydrogenation of maleic anhydride, maleic acid and related compounds to tetrahydrofuran, gamma-butyrolactone and 1,4-butanediol.

British Patent No. 1,534,232 teaches the hydrogenation of carboxylic acids, lactones or anhydrides using a hydrogenation catalyst consisting of palladium and rhenium on a carbon support. U.S. Patents 4,550,185 and 4,609,636 teach a process of making tetrahydrofuran and 1,4-butanediol by hydrogenating maleic acid, maleic anhydride or other hydrogenatable precursor in the presence of a catalyst comprising palladium and rhenium on a carbon support wherein the palladium and rhenium were present in the form of crystallites having an average palladium crystallite size of about 10 to 25 nm and an average rhenium crystallite size of less than 2.5 nm. The preparation of this catalyst is characterized by the deposition and reduction of the palladium species on the carbon support followed by the deposition and reduction of the rhenium species on the palladium impregnated carbon support. .

U.S. Patent 4,985,572 teaches a process for the catalytic hydrogenation of a carboxylic acid or an anhydride thereof to the corresponding alcohol and/or carboxylic acid ester using a catalyst comprising rhenium, palladium and at least one other metal capable of alloying with the palladium, all on a carbon support. The preferred metal capable of alloying with the palladium is silver but gold, copper, nickel, rhodium, tin, cobalt, aluminum, manganese, gallium, iron, chromium, and platinum also are taught. The preparation of this catalyst is characterized by the simultaneous deposition of palladium and silver on the carbon support followed by a high temperature (600°C) heat treatment. Rhenium is then deposited on the palladium/alloying metal impregnated carbon support. The resulting catalyst is then reduced.

WO 92/02298 discloses a hydrogenation catalyst comprising palladium and rhenium and one or more metals selected from the group consisting of rhodium, cobalt, platinum, ruthenium, iron, thulium, cerium, yttrium, neodymium, aluminum, praseodymium, holmium, hafnium, manganese, vanadium, chromium, gold, terbium, lutetium, nickel, scandium and niobium, on a support.

Generally, in the hydrogenation of maleic acid, maleic anhydride or other hydrogenatable precursor the above discussed catalysts have the propensity to produce more tetrahydrofuran and gamma-butyrolactone than 1,4-butanediol. An object of this invention is a process and a catalyst which will maximize 1,4-butanediol production and minimize gamma-butyrolactone production.

European Patent 848991 discloses an improved metal supported carbon catalyst for the hydrogenation of maleic acid, maleic anhydride or other hydrogenatable precursors to 1,4-butanediol and tetrahydrofuran. The catalyst has as essential metal components palladium, silver, rhenium and iron.

### SUMMARY OF THE INVENTION

The instant invention is a catalyst consisting of palladium, silver, rhenium and at least one of aluminum, cobalt and mixtures thereof, all on a carbon support and the use of this catalyst in a process for the production of 1,4-butanediol comprising catalytically hydrogenating a hydrogenatable precursor in contact with a hydrogen-containing gas.

Another embodiment of the instant invention is a method for making such catalyst for the production of 1,4-butanediol comprising:
(i) oxidizing a carbon support by contacting the carbon support with an oxidizing agent;
(ii) impregnating in one or more impregnation steps comprising contacting a carbon support with a source of palladium, silver, rhenium and at least one of aluminum, cobalt and mixtures thereof;
(iii) drying the impregnated carbon support to remove solvent after each impregnation step; and
(iv) heating the impregnated carbon support from ambient temperature to a temperature of between about 100° C and about 350°C under reducing conditions.

### DETAILED DESCRIPTION OF THE INVENTION

A catalyst consisting of palladium, rhenium, silver and at least one of aluminum, cobalt and mixtures thereof, all on a carbon support is employed in the hydrogenation of a hydrogenatable precursor to provide high yields of 1,4-butanediol and smaller yields of tetrahydrofuran with minimal gamma-butyrolactone formation.

### The Reactants

In the process of the instant invention, at least one hydrogenatable precursor is reacted with a hydrogen containing gas in the presence of the catalyst. As used herein a "hydrogenatable precursor" is any carboxylic acid or anhydride thereof, carboxylic acid ester, lactone or mixture thereof which when hydrogenated produces 1,4-butanediol. Representative hydrogenatable precursors include maleic acid, maleic anhydride, fumaric acid, succinic anhydride, succinic acid, succinate esters such as dimethyl succinate, maleate esters such as dimethyl maleate, gamma-butyrolactone or mixtures thereof. The preferred hydrogenatable precursors are maleic acid, maleic anhydride, succinic acid, succinic anhydride or mixtures thereof.

The most preferred hydrogenatable precursor is maleic acid which is typically obtained by reacting n-butane or benzene in an oxygen-containing gas in the presence of a catalyst to oxidize in the vapor phase the n-butane or benzene to maleic anhydride, and then collecting the maleic anhydride by a water quench to produce maleic acid in an aqueous solution. The oxidation of n-butane or benzene is typically operated at a temperature of about 300°C to 600°C and a pressure of about 0.5 to 20 atmospheres (50 to 2000 kPa).

Typically, the hydrogen (H₂) containing gas is commercially pure hydrogen with no diluent gases. However, the hydrogen containing gas in addition to hydrogen (H₂) may also contain nitrogen (N₂), any gaseous hydrocarbon (e.g. methane), as well as gaseous oxides of carbon, (e.g. carbon monoxide, carbon dioxide).

### The Catalyst

The catalyst employed in the instant invention consists essentially of palladium, silver, rhenium and at least one of aluminum, cobalt and mixtures thereof, all supported on carbon. The carbons for use in this invention have a BET surface area of at least 200 m²/g, and preferably be in the range of 500-1500 m²/g.

The catalyst composition comprises about 0.1 to about 20 weight percent palladium, preferably about 2 to about 8 weight percent palladium, more preferably about 2 to about 4 weight percent palladium; about 0.1 to about 20 weight percent silver, preferably about 1 to about 8 weight percent silver, more preferably about 2 to about 4 weight percent silver; about 0.1 to about 20 weight percent rhenium, preferably about 1 to about 10 weight percent rhenium, more preferably about 5 to about 9 weight percent rhenium; and about 0.01 to about 10 weight percent of at least one of, aluminum, cobalt and mixtures thereof, preferably about 0.1 to about 5 weight percent of at least one of aluminum, cobalt and mixtures thereof, more preferably about 0.2 to about 0.6 weight percent of at least one of aluminum, cobalt and mixtures thereof. The ratio of palladium to silver is between 10 to 1 and 1 to 10.

The catalysts of this invention may be conveniently prepared by oxidizing the carbon support followed by impregnation of the carbon support, either in single or multiple impregnation steps, with a solution or solutions containing at least one palladium, silver, rhenium or at least one of aluminum, or cobalt compound.

Preferably, the carbon support is first oxidized by contacting the carbon support, prior to deposition of the metals, with a oxidizing agent. Catalysts prepared in this manner show a dramatic improvement in activity and selectivity over catalysts prepared with non-oxidized carbon support. A number of oxidizing agents such as nitric acid, hydrogen peroxide, sodium hypochlorite, ammonium persulfate, perchloric acid, and oxygen may be effective in this process. Liquid phase oxidizing agents are preferred. Nitric acid at elevated temperatures has been found to be especially effective for this procedure. Gaseous phase oxidizing agents include any oxygen-containing gas, e.g. air. Gaseous oxidizing agents are contacted with the carbon support at temperatures of about 200°C or greater and at pressures of about atmospheric or greater.

As stated earlier, the catalysts of this invention are prepared by impregnation of the carbon support, either in single or multiple impregnation steps, with a solution or solutions containing at least one palladium, silver, rhenium, aluminum or cobalt compound. As used herein, impregnation of the carbon support means to cause the carbon support to be filled, imbued, permeated, saturated or coated. The impregnating solution may optionally contain complexing agents to help solubilize one or more of the metal compounds. The impregnating solution may also optionally be combined with the oxidizing agent prior to or in situ with contacting the carbon support. The catalyst is dried after each impregnation step to remove any carrier solvent. Drying temperatures are between about 80°C and about 150°C.

The solutions of palladium compound, silver compound, rhenium compound, aluminum compound, cobalt compound or mixtures thereof can be applied to the carbon by immersing or suspending the support material in the solution or by spraying the solution onto the carbon. The solution containing the palladium compound is typically an aqueous solution containing an amount of palladium compound to yield a catalyst product with the requisite amount of palladium. The palladium compound may be palladium nitrate or a palladium compound such as a chloride, carbonate, carboxylate, acetate, acetyl acetonate, or amine. The solution containing the silver compound is typically an aqueous one containing an amount of silver compound to yield a catalyst product with the requisite amount of silver. The palladium and silver compounds should be thermally decomposable and reducible to the metals. The solution containing the rhenium compound is typically an aqueous one containing an amount of rhenium compound to yield a catalyst product with the requisite amount of rhenium. The rhenium compound is typically perrhenic acid, ammonium perrhenate or an alkali metal perrhenate.

The solution containing the aluminum compound is typically an aqueous one containing an amount of aluminum compound to yield a catalyst product with the requisite amount of aluminum. The aluminum compound is typically aluminum nitrate, but other suitable aluminum containing compounds include, but are not limited to, aluminum acetate and aluminum chloride. The solution containing the cobalt compound is typically an aqueous one containing an amount of cobalt compound to yield a catalyst product with the requisite amount of cobalt. The cobalt compound is typically cobalt nitrate, but other suitable cobalt containing compounds include, but are not limited to, cobalt acetate, cobalt chloride, cobalt maleate, and cobalt fumarate.

The impregnating solution(s) may optionally contain metal complexing agents to help solubilize one or more of the metal compounds. The addition of acetonitrile to the impregnating solution allows the Pd, Ag, and Re compounds be added in a single step. Nitric acid or other oxidizing agent may also be added to the impregnating solution.

After impregnation with palladium, silver, rhenium and at least one of aluminum, cobalt and mixtures thereof and then drying the impregnated carbon support, the catalyst is activated by heating the impregnated carbon support under reducing conditions from ambient temperature (i.e. typically room temperature) to a temperature of between about 120°C and 350°C, preferably between about 150°C and about 300°C. Hydrogen, or a mixture of hydrogen and nitrogen, in contact with the catalyst may be conveniently used for the catalyst reduction. Reduction of the impregnated carbon support is only after the carbon support has been impregnated with palladium, silver, rhenium and at least one of aluminum, cobalt and mixtures thereof In the case of multiple impregnation steps and multiple dryings, the reduction of the catalyst is done after the final drying.

After step (iv) of the method of making the catalyst, the catalyst may be contacted with a hydrogenatable precusor and hydrogen and heated from ambient temperature to between about 50°C and about 250°C.

The palladium in catalysts of the present invention is present in the form of crystallites having an average crystallite size of less than 100 angstroms (10 nm). More specifically, when freshly reduced samples of the palladium/silver/rhenium on a carbon support as used herein are analyzed by X-ray diffraction (XRD) and Scanning Transmission Electron Microscopy (STEM), the palladium containing particles (i.e. particles of palladium, particles of palladium and silver, or particles of palladium and rhenium) in the catalyst are finely dispersed and have a mean crystallite size of less than about 50 angstroms (5 nm). As used herein the "particle size distribution" and "mean particle size" are as defined in "Structure of Metal Catalysts" by J. R. Anderson, pages 358-359, Academic Press (1975), which is incorporated herein by reference.

Lastly the preparation of the catalysts described herein does not employ large amounts of excess water which must be removed during the drying step nor does it employ a high temperature (i.e. about 600°C) treatment step as taught in U.S. Patent 4,985,572.

Upon completion of the catalyst preparation described herein, aluminum, or cobalt are present in the catalyst. However, during the hydrogenation of maleic acid and depending upon the conditions in the hydrogenation reactor, some aluminum or cobalt may be leached from the catalyst.

### The process

The method for carrying out the process comprises reacting a hydrogenatable precursor with a hydrogen-containing gas in the presence of the hydrogenation catalyst, and recovering and purifying the reaction products by distillation.

The liquid phase hydrogenation of this invention can be run using conventional apparatus and techniques in a stirred-tank reactor or in a fixed-bed reactor. Single or multiple-stage reactors may be employed. The amount of catalyst required will vary widely and is dependent upon a number of factors such as reactor size and design, contact time and the like.

The hydrogen-containing gas is fed continuously, generally with the hydrogen in considerable stoichiometric excess to the other reactants. Unreacted hydrogen can be returned to the reactor as a recycle stream. The precursor solution, e.g., maleic acid solution, is fed continuously at concentrations ranging from dilute solutions to near the maximum solubility level, typically about 30 to about 50 weight percent.

Preferably the hydrogenation step is run at a temperature of about 50°C to 350°C, and a hydrogen pressure of about 20-400 atmospheres with hydrogen to hydrogenatable precursor ratios (H₂/P) of between 5 to 1 and 1000 to 1 and contact times of 0.1 minute to 20 hours. For maximum 1,4-butanediol production the reaction temperature is between about 50°C and 250°C and more preferably between about 80°C and 200°C.

The reaction products, 1,4-butanediol, tetrahydrofuran, gamma-butyrolactone or mixtures thereof, are advantageously separated by fractional distillation. By-products which are formed in small amounts or unreacted feed, such as for example, succinic anhydride or succinic acid, are optionally returned to the hydrogenation stage. The gamma-butyrolactone may also be recycled to the hydrogenation reactor.

Using the process of this invention, more specifically using the hydrogenation catalyst described herein, maleic acid is converted virtually quantitatively in a simple reaction. The yields of 1,4-butanediol and tetrahydrofuran achieved are about 80 mole percent or greater, typically about 90 mole percent or greater, with a majority portion of the yield being 1,4-butanediol. Reaction by-products may include n-butanol, n-butyric acid, n-propanol, propionic acid, methane, propane, n-butane, carbon monoxide, and carbon dioxide. However, the formation of non-utilizable by-products is slight.

### SPECIFIC EMBODIMENTS

In order to illustrate the instant invention the following examples are provided.

### Comparative Example A: Preparation of PdAgRe on carbon

45g of concentrated nitric acid (70 wt%) was diluted to 50 cc with deionized water. This solution was used to impregnate 74.6g of CECA 1.5 mm ACL40 carbon extrudate. During the impregnation the flask was occasionally cooled. The mixture was allowed to stand for 80 minutes, and then dried at 130°C for 3h. This procedure was repeated with a 35 minute standing time and a 16h drying time.

35.1g of palladium nitrate solution (8.5 wt% Pd), 11.95g of perrhenic acid (53.3 wt% Re) solution and 7.9g of concentrated (70 wt% nitric acid), were diluted to 50 cc with deionized water. The ACL40 was then gradually impregnated with the Pd+Re solution. The flask was occasionally cooled during the impregnation. The mixture was allowed to stand for 2h, and then dried at 130°C for 2h.

4.7g of silver nitrate and 7.9g of concentrated nitric acid were diluted to 50 cc with deionized water. The PdRe/ACL40 was then gradually impregnated with the silver nitrate solution with occasional cooling of the flask. The mixture was allowed to stand for 3.5h, and then dried at 130C for 64h. The resulting catalyst was 3.3 wt% Pd/ 3.3 wt% Ag/ 7.1 wt% Re.

### Comparative Example 1: Preparation of PdAgReFe on carbon

45g of concentrated nitric acid (70 wt%) and 1g of ferric nitrate (Fe(NO₃)₃.9H₂O) were diluted to 50 cc with deionized water. This solution was used to impregnate 74.6g of CECA 1.5 mm ACL40 carbon extrudate. During the impregnation the flask was occasionally cooled. The mixture was allowed to stand for 65 minutes, and then dried at 130°C for 2h. This procedure was repeated with a 65 minute standing time and a 2.4h drying time.

35.1 of palladium nitrate solution (8.5 wt% Pd), 11.95g of perrhenic acid (53.3 wt% Re) solution, 7.9g of concentrated nitric acid (70 wt%), were diluted to 50 cc with deionized water. The ACL40 was then gradually impregnated with the Pd/Re solution. The flask was occasionally cooled during the impregnation. The mixture was allowed to stand for 2.5h, and then dried at 130°C for 2.25h.

4.7g of silver nitrate and 7.9g of concentrated nitric acid were diluted to 50 cc with deionized water. The PdRe/ACL40 was then gradually impregnated with the silver nitrate solution with occasional cooling of the flask. The mixture was allowed to stand for 80 minutes, and then dried at 130°C for 69h. The resulting catalyst was 3.3 wt% Pd/ 3.3 wt% Ag/ 7.1 wt% Re/ 0.3 wt% Fe.

### Example 2: Hydrogenation of Aqueous Maleic Acid and Catalyst Testing

The catalyst of Comparative Example A and Example 1 were each tested in two Hastelloy C276 reactors connected in series using heated Hastelloy C276 tubing. The reactors had an internal diameter of 0.516", and each was fitted with a 1/8" axial Hastelloy C276 thermowell.

Each catalyst was mixed with 50/70 mesh quartz chips (0.625g quartz per g of catalyst) before charging to the reactor. 20 cc (12.15g) of catalyst was placed in the first reactor, and 40 cc (24.3g) in the second reactor. Prior to testing the catalyst was reduced at atmospheric pressure in flowing hydrogen (400 sccm) with the following temperature ramp:
Room Temperature to 30°C over 5 hours
30°C to 100°C over 2 hours
100°C to 230°C over 11 hours
maintain 230°C for 5 hours
The reactors were operated with hydrogen recycle. A small portion of the hydrogen was vented to prevent the accumulation of non-condensable gases. The maleic acid concentration in the liquid feed was 35.5 wt%. The process conditions for the catalyst testing were as following process conditions:
- Pressure: 2500 psig
- H₂/Maleic Acid Feed Ratio: 88
- H₂ Make-up to Recycle Ratio: 0.083
- First Reactor:
   Average Set Temperature: 100°C
   LHSV; 1.6h⁻¹
- Second Reactor:
   Average Set Temperature: 153-162°C
   LHSV: 0.8h⁻¹

Table 1 summarizes the results of the testing on the PdAgRe/C and PdAgReFe/C catalysts. Product selectivity's were calculated on a molar C₄ basis.

**Table 1 - Catalyst Performance Data**

| Catalyst | Hrs. on Stream | Temp. (C) | %BDO sel | %THF sel | %GBL sel | %BuOH sel | %SAC sel |
|---|---|---|---|---|---|---|---|
| PdAgReFe/C (Ex. 1) 2 Comp. | 185 | 153 | 89.5 | 5.6 | 0.6 | 4.0 | 0.05 |
| PdAgRe/C (Comp. Ex A) | 185 | 162 | 86.3 | 8.8 | 0.9 | 3.8 | 0.08 |

Table 1 illustrates that the BDO yield is significantly higher for PdAgReFe/C. Table 1 also illustrates that the iron containing catalyst (Comparative Example 1) is more active than the non iron containing species (Comparative Example A). This is evidenced by better overall conversions at the lower reaction temperature.

### Example 3: Preparation of PdAgReM on Carbon, where M is Fe (Comparative), Al, or Co

### a) Preparation of Precursor PdRe/Norit RX1.5 Extra

584g of Norit RX1.5 Extra carbon extrudate (acquired from Norit Americas Inc. located in Atlanta, Georgia) was impregnated with 719g of concentrated nitric acid (70 wt%). The material was allowed to stand for 90 minutes, and was then dried in an oven at 130°C overnight.

218.1g of palladium nitrate solution (8.5 wt% Pd in 10 wt% HNO₃), 114g of perrhenic acid (56.36 wt% Re), 234.6g of concentrated nitric acid, and 151g of de-ionized water were mixed together. The carbon was impregnated with 96% of the Pd/Re solution, and the mixture allowed to stand for 2h. After drying overnight at 130°C, 671.7g of material was obtained, with a Pd content of 2.6 wt%, and Re content of 9.2 wt%. The moisture content (%wt loss at 150 °C) was 3.4 wt%. The material was riffled into eight ~84g portions.

### b) Preparation of PdAgReM/Norit RX1.5 Extra

Three catalysts were made as described below, with M = Fe (Comparative), Al or Co. The Table below summarizes the materials used for the different catalyst preparations:

**Table 2**

| **PdRe/C Riffle Lot #: Wt (g)** | **Wt. AgNO**_{**3**} **(g)** | **Wt. HNO**_{**3**} **(70 wt%)** | **Wt. H**_{**2**}**O (g)** | **M** | **Metal Nitrate Form** | **Weight of Metal Nitrate (g)** |
|---|---|---|---|---|---|---|
| 63.4g | 2.5 | 6.9 | 38 | Fe | Fe(NO₃)₃·9H₂O | 2 |
| 63.4g | 2.5 | 6.9 | 38 | Al | Al(NO₃)₃·9H₂O | 1.86 |
| 63.4g | 2.5 | 6.9 | 38 | Co | Co(NO₃)₂-6H₂0 | 1.44 |

The preparation for M=Fe is as follows: 2.5g of silver nitrate, 6.9g of concentrated nitric acid, and 2g of Fe(NO₃)₃·9H₂O, were added to 38g of deionized water, and the mixture stirred to dissolve the solids. 63.4g (140cc) of PdRe/Norit (Riffle Lot 1) was then impregnated with the Ag/Fe solution, and the mixture allowed to stand for about 2.5 h. The material was then place in an oven at 130C, and dried for 4.5h. The other catalysts' (M= Al or Co) were prepared in a similar manner.

### Example 4: Hydrogenation of Aqueous Maleic Acid PdAgReM on Carbon Catalyst, where M is Fe (Comparative), Al, or Co

Catalyst testing was carried out using two Hasteloy C276 reactors connected in series using heated Hasteloy C276 tubing. The reactors had an internal diameter of 0.516", and each was fitted with a 1/8" axial Hasteloy C276 thermowell.

The catalyst was mixed with 50/70 mesh quartz chips (0.625g quartz per g of catalyst) before charging to the reactor. 20 cc (12.15g) of catalyst was placed in the first reactor, and 40cc (24.3g) in the second reactor. Prior to testing the catalyst was reduced at atmospheric pressure in flowing hydrogen (400 sccm) with the following temperature ramp:
RT to 30C in 5h
30-100C in 2h
100-230C in 11h
@230C for 5h

The reactors were operated with hydrogen recycle. A small portion of the hydrogen was vented to prevent the accumulation of non-condensable gases. The maleic acid concentration in the aqueous liquid feed was 35.5 wt%. The process conditions for the catalyst testing were as follows:
- Pressure: 2500 psig
- H₂/(MAC+FAC) Feed Ratio: 88
- H₂ Make-up to Recycle Ratio: 0.083
- First Reactor:
   Average Set Temperature: 110C
   LHSV: 1.6 h⁻¹
- Second Reactor:
   Average Set Temperature: 153-162C
   LHSV: 0.8 h⁻¹

Table 3 summarizes the results of the testing for the PdAgReM/Norit RX1.5 Extra catalysts.

**Table 3 Catalyst Performance Data**

| **Catalyst** | **TOS** | **Set T** | **Mass** | **BDO** | **THF** | **GBL** | **BuOH** | **PrOH** | **SAC** |
|---|---|---|---|---|---|---|---|---|---|
| | | | **Bal** | **sel** | **sel** | **sel** | **sel** | **sel** | **sel** |
| PdAgReAl/C | 14.3 | 165 | 0.97 | 88.15 | 6.51 | 0.22 | 4.30 | 0.42 | 0.03 |
| | 31.8 | 161 | 1.00 | 89.18 | 6.81 | 0.20 | 3.01 | 0.30 | 0.04 |
| | 44.4 | 152 | 0.99 | 91.01 | 4.97 | 1.43 | 1.80 | 0.17 | 0.28 |
| PdAgReCo/C | 19.1 | 165 | 0.97 | 86.92 | 8.07 | 0.22 | 4.12 | 0.55 | 0.02 |
| | 44.5 | 152 | 0.97 | 88.83 | 6.67 | 1.82 | 1.95 | 0.19 | 0.39 |
| | 68.7 | 152 | 0.99 | 87.78 | 7.34 | 2.12 | 1.96 | 0.19 | 0.46 |
| PdAgReFe/C | 19.0 | 165 | 0.98 | 88.70 | 7.82 | 0.23 | 2.79 | 0.32 | 0.01 |
| (Comparative) | 43.1 | 152 | 0.99 | 87.94 | 6.39 | 3.10 | 1.59 | 0.15 | 0.70 |
| | 64.3 | 155 | 1.00 | 88.62 | 7.78 | 1.15 | 1.93 | 0.19 | 0.21 |

| | | | | | | | | | |
|---|---|---|---|---|---|---|---|---|---|
| TOS = Time on Stream (hrs.) Set T = Reactor Set Temperature (°C) Mass Bal = Mass Balance BDO Sel = % Selectivity to 1,4-Butanediol THF sel = % Selectivity to Tetrahydrofuran GBL sel = % Selectivity to GammabutyroTactone BuOH sel = % Selectivity to Butanol PrOH sel = % Selectivity to Propanol SAC sel = % Selectivity to Succinic Acid | | | | | | | | | |

It is to be understood that the subject invention is not to be limited by the examples set forth herein. These have been provided merely to demonstrate operability, and the selection of catalysts, metal sources, carbon supports, concentrations, contact times, solids loadings, feedstocks, reaction conditions, and products, if any, can be determined from the total specification disclosure provided, without departing from the scope of the invention as defined in the attached claims.

## Claims

1. A catalyst consisting essentially of palladium, silver, rhenium and a metal selected from aluminum, cobalt and mixtures thereof on a carbon support wherein the catalyst comprises between 0.1 to 20 wt % palladium, between 0.1 to 20 wt % silver, between 0.1 to 20 wt % rhenium, and between 0.1 to 5 wt % of a metal selected from aluminum, cobalt and mixtures thereof.

2. The catalyst of claim 1 wherein the catalyst comprises 2 to 4 wt % palladium, 2 to 4 wt % silver, 5 to 9 wt % rhenium, and 0.2 to 0.6 wt% of a metal selected from aluminum, cobalt and mixtures thereof.

3. A process for the production of 1,4-butanediol comprising catalytically hydrogenating a hydrogenatable precursor selected from the group consisting of maleic acid, maleic anhydride, fumaric acid, succinic acid, succinic anhydride, maleate esters, succinate esters, gamma-butyrolactone and mixtures thereof, in contact with a hydrogen-containing gas and a hydrogenation catalyst comprising palladium, silver, rhenium and iron on a carbon support wherein the catalyst comprises between 0.1 to 20 wt % palladium, between 0.1 to 20 wt % silver, between 0.1 to 20 wt % rhenium, and between 0.1 to 5 wt % of a metal selected from aluminum, cobalt and mixtures thereof and wherein said process is conducted at a temperature of 50° C to 350° C, a hydrogen-containing gas pressure of between 20 and 400 atmospheres, a ratio of hydrogen to hydrogenatable precursor of between 5 to 1 and 1000 to 1, and a contact time of between 0.1 minute and 20 hours.

4. A process as claimed in claim 3 wherein the hydrogenatable precursor is at least one of maleic acid, succinic acid, or gamma-butyrolactone.

5. A process as claimed in claim 3 or claim 4 wherein the catalyst comprises 2 to 4 wt % palladium, 2 to 4 wt % silver, 5 to 9 wt % rhenium, and 0.2 to 0.6 wt % of a metal selected from aluminum, cobalt and mixtures thereof.

6. A method for making the catalyst as claimed in claims 1 or 2 or the catalyst for use in the process of any one of claims 3 to 5 comprising:
(i) oxidizing a carbon support by contacting the carbon support with an oxidizing agent;
(ii) impregnating in one or more impregnation steps comprising contacting a carbon support with a source of palladium, silver, rhenium and a metal selected from aluminum, cobalt and mixtures thereof;
(iii) drying the impregnated carbon support to remove solvent after each impregnation step; and
(iv) heating the impregnated carbon support from ambient temperature to a temperature of between 100°C and 350°C under reducing conditions.

7. A method as claimed in claim 6 wherein the carbon support is contacted with an oxidizing agent at approximately the same time as the impregnation of the carbon support with the source of the palladium, silver, rhenium and a metal selected from aluminum, cobalt and mixtures thereof.

8. A method as claimed in claim 6 or claim 7 wherein the oxidizing agent is selected from the group consisting of nitric acid, hydrogen peroxide, sodium hypochlorite, ammonium persulfate, perchloric acid, and oxygen.

9. A method as claimed in any one of claims 6 to 8 wherein after step (iv) the catalyst is contacted with a hydrogenatable precursor selected from the group consisting of maleic acid, maleic anhydride, fumaric acid, succinic acid, succinic anhydride, dimethyl succinate, gamma-butyrolactone and mixtures thereof, and hydrogen and heated from ambient temperature to between 50°C and 250°C.

10. A process for the production of tetrahydrofuran and 1,4-butanediol comprising catalytically hydrogenating a hydrogenatable precursor selected from the group consisting of maleic acid, maleic anhydride, fumaric acid, succinic acid, succinic anhydride, dimethyl succinate, gamma-butyrolactone and mixtures thereof in contact with a catalyst according to claim 1 or claim 2 as prepared by the method of any one of claims 6 to 9 and wherein said process is conducted at a temperature of 50° C to 350° C, a hydrogen-containing gas pressure of between 20 and 400 atmospheres, a ratio of hydrogen to hydrogenatable precursor of between 5 to 1 and 1000 to 1, and a contact time of between 0.1 minute and 20 hours.

11. A process as claimed in claim 10 wherein the hydrogenatable precursor is at least one of maleic acid, succinic acid, or gamma-butyrolactone.

12. A process as claimed in claim 10 or claim 11 wherein the catalyst comprises 2 to 4 wt % palladium, 2 to 4 wt % silver, 5 to 9 wt % rhenium, and 0.2 to 0.6 wt % of a metal selected from aluminum, cobalt and mixtures thereof.

13. A process as claimed in any one of claims 10 to 12 wherein the oxidizing agent is selected from the group consisting of nitric acid, hydrogen peroxide, sodium hypochlorite, ammonium persulfate, perchloric acid and oxygen.

## Patentansprüche

1. Katalysator, bestehend im wesentlichen aus Palladium, Silber, Rhenium und einem Metall, ausgewählt aus Aluminium, Kobalt und Gemischen davon, auf einem Kohlenstoffträger, wobei der Katalysator zwischen 0,1 und 20 Gew.-% Palladium, zwischen 0,1 und 20 Gew.-% Silber, zwischen 0,1 und 20 Gew.-% Rhenium und zwischen 0,1 und 5 Gew.-% eines Metalls, ausgewählt aus Aluminium, Kobalt und Gemischen davon, umfaßt.

2. Katalysator nach Anspruch 1, wobei der Katalysator 2 bis 4 Gew.-% Palladium, 2 bis 4 Gew.-% Silber, 5 bis 9 Gew.-% Rhenium und 0,2 bis 0,6 Gew.-% eines Metalls, ausgewählt aus Aluminium, Kobalt und Gemischen davon, umfaßt.

3. Verfahren zur Herstellung von 1,4-Butandiol, umfassend katalytische Hydrierung eines hydrierbaren Präkursors, ausgewählt aus der Gruppe, bestehend aus Maleinsäure, Maleinsäureanhydrid, Fumarsäure, Bernsteinsäure, Bernsteinsäureanhydrid, Maleatestern, Succinatestem, gamma-Butyrolacton und Gemischen davon, im Kontakt mit einem Wasserstoffenthaltenden Gas und einem Hydrierungskatalysator, umfassend Palladium, Silber, Rhenium und Eisen auf einem Kohlenstoffträger, wobei der Katalysator zwischen 0,1 und 20 Gew.-% Palladium, zwischen 0,1 und 20 Gew.-% Silber, zwischen 0,1 und 20 Gew.-% Rhenium und zwischen 0.1 und 5 Gew.-% eines Metalls, ausgewählt aus Aluminium, Kobalt und Gemischen davon, umfaßt, und wobei das Verfahren bei einer Temperatur von 50 bis 350 °C, einem Druck des Wasserstoff-enthaltendes Gases zwischen 20 und 400 Atmosphären, einem Verhältnis von Wasserstoff zu hydrierbaren Präkursor zwischen 5 zu 1 und 1000 zu 1 und einer Kontaktzeit zwischen 0,1 Minuten und 20 Stunden durchgeführt wird.

4. Verfahren nach Anspruch 3, wobei der hydrierbare Präkursor mindestens eines von Maleinsäure, Bernsteinsäure oder gamma-Butyrolacton ist.

5. Verfahren nach Anspruch 3 oder Anspruch 4, wobei der Katalysator 2 bis 4 Gew.-% Palladium, 2 bis 4 Gew.-% Silber, 5 bis 9 Gew.-% Rhenium und 0,2 bis 0,6 Gew.-% eines Metalls, ausgewählt aus Aluminium, Kobalt und Gemischen davon, umfaßt.

6. Verfahren zur Herstellung des Katalysators nach den Ansprüchen 1 oder 2 oder des Katalysators zur Verwendung in dem Verfahren nach einem der Ansprüche 3 bis 5, umfassend:
(i) Oxidieren eines Kohlenstoffträgers durch Kontaktieren des Kohlenstoffträgers mit einem Oxidationsmittel;
(ii) Imprägnieren in einem oder mehreren Imprägnierschritten, umfassend das Kontaktieren eines Kohlenstoffträgers mit einer Quelle an Palladium, Silber, Rhenium und einem Metall, ausgewählt aus Aluminium, Kobalt und Gemischen davon;
(iii) Trocknen des imprägnierten Kohlenstoffträgers zur Entfernung des Lösungsmittels nach jedem Imprägnierungsschritt; und
(iv) Erhitzen des imprägnierten Kohlenstoffträgers von Umgebungstemperatur auf eine Temperatur zwischen 100 °C und 350 °C unter Reduktionsbedingungen.

7. Verfahren nach Anspruch 6, wobei der Kohlenstoffträger mit einem Oxidationsmittel zu ungefähr derselben Zeit wie der Imprägnierung des Kohlenstoffträgers mit der Quelle an Palladium, Silber, Rhenium und einem Metall, ausgewählt aus Aluminium, Kobalt und Gemischen davon, kontaktiert wird.

8. Verfahren nach Anspruch 6 oder Anspruch 7, wobei das Oxidationsmittel aus der Gruppe ausgewählt wird, bestehend aus Salpetersäure, Wasserstoffperoxid, Natriumhypochlorit, Ammoniumpersulfat, Perchlorsäure und Sauerstoff.

9. Verfahren nach einem der Ansprüche 6 bis 8, wobei nach Schritt (iv) der Katalysator mit einem hydrierbaren Präkursor, ausgewählt aus der Gruppe, bestehend aus Maleinsäure, Maleinsäureanhydrid, Fumarsäure, Bernsteinsäure, Bernsteinsäureanhydrid, Dimethylsuccinat, gamma-Butyrolacton und Gemischen davon, und Wasserstoff kontaktiert und von Umgebungstemperatur auf zwischen 50 und 250 °C erhitzt wird.

10. Verfahren zur Herstellung von Tetrahydrofuran und 1,4-Butandiol, umfassend die katalytische Hydrierung eines hydrierbaren Präkursors, ausgewählt aus der Gruppe, bestehend aus Maleinsäure, Maleinsäureanhydrid, Fumarsäure, Bernsteinsäure, Bernsteinsäureanhydrid, Dimethylsuccinat, gamma-Butyrolacton und Gemischen davon, im Kontakt mit einem Katalysator nach Anspruch 1 oder Anspruch 2, wie durch das Verfahren nach einem der Ansprüche 6 bis 9 hergestellt, und wobei das Verfahren bei einer Temperatur von 50 bis 350 °C, einem Druck des Wasserstoff-enthaltendes Gases zwischen 20 und 400 Atmosphären, einem Verhältnis von Wasserstoff zu hydrierbaren Präkursor zwischen 5 zu 1 und 1000 zu 1 und einer Kontaktzeit zwischen 0,1 Minuten und 20 Stunden durchgeführt wird.

11. Verfahren nach Anspruch 10, wobei der hydrierbare Präkursor mindestens eines von Maleinsäure, Bernsteinsäure oder gamma-Butyrolacton ist.

12. Verfahren nach Anspruch 10 oder Anspruch 11, wobei der Katalysator 2 bis 4 Gew.-% Palladium, 2 bis 4 Gew.-% Silber, 5 bis 9 Gew.-% Rhenium und 0,2 bis 0,6 Gew.-% eines Metalls, ausgewählt aus Aluminium, Kobalt und Gemischen davon, umfaßt.

13. Verfahren nach einem der Ansprüche 10 bis 12, wobei das Oxidationsmittel aus der Gruppe ausgewählt wird, bestehend aus Salpetersäure, Wasserstoffperoxid, Natriumhypochlorit, Ammoniumpersulfat, Perchlorsäure und Sauerstoff.

## Revendications

1. Catalyseur essentiellement constitué de palladium, d'argent, de rhénium et d'un métal choisi parmi l'aluminium, le cobalt et des mélanges de ceux-ci sur un support de carbone, dans lequel le catalyseur comprend entre 0,1 et 20 % en poids de palladium, entre 0,1 et 20 % en poids d'argent, entre 0,1 et 20 % en poids de rhénium, et entre 0,1 et 5 % en poids d'un métal choisi parmi l'aluminium, le cobalt et des mélanges de ceux-ci.

2. Catalyseur selon la revendication 1, dans lequel le catalyseur comprend 2 à 4 % en poids de palladium, 2 à 4 % en poids d'argent, 5 à 9 % en poids de rhénium, et 0,2 à 0,6 % en poids d'un métal choisi parmi l'aluminium, le cobalt et des mélanges de ceux-ci.

3. Procédé de production de 1,4-butanediol comprenant l'hydrogénation catalytique d'un précurseur hydrogénable choisi dans le groupe constitué par l'acide maléique, l'anhydride maléique, l'acide fumarique, l'acide succinique, l'anhydride succinique, les esters de maléate, les esters de succinate, la gamma-butyrolactone et des mélanges de ceux-ci, en contact avec un gaz contenant de l'hydrogène et un catalyseur d'hydrogénation comprenant le palladium, l'argent, le rhénium et le fer sur un support de carbone, dans lequel le catalyseur comprend entre 0,1 et 20 % en poids de palladium, entre 0,1 et 20 % en poids d'argent, entre 0,1 et 20 % en poids de rhénium, et entre 0,1 et 5 % en poids d'un métal choisi parmi l'aluminium, le cobalt et des mélanges de ceux-ci, et dans lequel ledit procédé est réalisé à une température de 50°C à 350°C, une pression de gaz contenant de l'hydrogène comprise entre 20 et 400 atmosphères, un rapport de l'hydrogène au précurseur hydrogénable compris entre 5 à 1 et 1000 à 1, et une durée de contact comprise entre 0,1 minute et 20 heures.

4. Procédé selon la revendication 3, dans lequel le précurseur hydrogénable est au moins l'un de l'acide maléique, l'acide succinique ou la gamma-butyrolactone.

5. Procédé selon la revendication 3 ou la revendication 4, dans lequel le catalyseur comprend 2 à 4 % en poids de palladium, 2 à 4 % en poids d'argent, 5 à 9 % en poids de rhénium, et 0,2 à 0,6 % en poids d'un métal choisi parmi l'aluminium, le cobalt et des mélanges de ceux-ci.

6. Procédé de préparation du catalyseur selon les revendications 1 ou 2 ou catalyseur à utiliser dans le procédé selon l'une quelconque des revendications 3 à 5 comprenant :
(i) l'oxydation d'un support de carbone en mettant en contact le support de carbone avec un agent oxydant ;
(ii) l'imprégnation en une ou plusieurs étapes d'imprégnation comprenant la mise en contact d'un support de carbone avec une source de palladium, d'argent, de rhénium et d'un métal choisi parmi l'aluminium, le cobalt, et des mélanges de ceux-ci ;
(iii) le séchage du support de carbone imprégné pour éliminer le solvant après chaque étape d'imprégnation ; et
(iv) le chauffage du support de carbone imprégné de la température ambiante à une température comprise entre 100°C et 350°C dans des conditions réductrices.

7. Procédé selon la revendication 6, dans lequel le support de carbone est mis en contact avec un agent oxydant environ en même temps que l'imprégnation du support de carbone avec la source du palladium, de l'argent, du rhénium et d'un métal choisi parmi l'aluminium, le cobalt et des mélanges de ceux-ci.

8. Procédé selon la revendication 6 ou la revendication 7, dans lequel l'agent oxydant est choisi dans le groupe constitué par l'acide nitrique, le peroxyde d'hydrogène, l'hypochlorite de sodium, le persulfate d'ammonium, l'acide perchlorique et l'oxygène.

9. Procédé selon l'une quelconque des revendications 6 à 8, dans lequel, après l'étape (iv), le catalyseur est mis en contact avec un précurseur hydrogénable choisi dans le groupe constitué par l'acide maléique, l'anhydride maléique, l'acide fumarique, l'acide succinique, l'anhydride succinique, le diméthyl succinate, la gamma-butyrolactone et des mélanges de ceux-ci, et l'hydrogène, et chauffé de la température ambiante à une température comprise entre 50°C et 250°C.

10. Procédé de production de tétrahydrofurane et de 1,4-butanediol comprenant l'hydrogénation catalytique d'un précurseur hydrogénable choisi dans le groupe constitué par l'acide maléique, l'anhydride maléique, l'acide fumarique, l'acide succinique, l'anhydride succinique, le diméthyl succinate, la gamma-butyrolactone et des mélanges de ceux-ci, en contact avec un catalyseur selon la revendication 1 ou la revendication 2 comme préparé par le procédé selon l'une quelconque des revendications 6 à 9, et dans lequel ledit procédé est réalisé à une température de 50°C à 350°C, une pression de gaz contenant de l'hydrogène comprise entre 20 et 400 atmosphères, un rapport de l'hydrogène au précurseur hydrogénable compris entre 5 à 1 et 1 000 à 1, et une durée de contact comprise entre 0,1 minute et 20 heures.

11. Procédé selon la revendication 10, dans lequel le précurseur hydrogénable est au moins l'un de l'acide maléique, l'acide succinique ou la gamma-butyrolactone.

12. Procédé selon la revendication 10 ou la revendication 11, dans lequel le catalyseur comprend 2 à 4 % en poids de palladium, 2 à 4 % en poids d'argent, 5 à 9 % en poids de rhénium, et 0,2 à 0,6 % en poids d'un métal choisi parmi l'aluminium, le cobalt et des mélanges de ceux-ci.

13. Procédé selon l'une quelconque des revendications 10 à 12, dans lequel l'agent oxydant est choisi dans le groupe constitué par l'acide nitrique, le peroxyde d'hydrogène, l'hypochlorite de sodium, le persulfate d'ammonium, l'acide perchlorique et l'oxygène.
